# EUROPÄISCHE PATENTSCHRIFT

(11) **EP 1 959 868 B1**
(45) Veröffentlichungstag und Bekanntmachung des Hinweises auf die Patenterteilung: **20.01.2010**
(21) Anmeldenummer: 06819734.2
(22) Anmeldetag: 23.11.2006
(51) Int. Cl.: A61F 2/30, A61B 19/00

(54) **VERFAHREN UND VORRICHTUNG ZUM PRÄPARIEREN EINES IMPLANTATS AUS EINEM IMPLANTATMATERIAL**
METHOD AND DEVICE FOR PREPARING AN IMPLANT FROM AN IMPLANT MATERIAL
PROCEDE ET DISPOSITIF DE PREPARATION D'UN IMPLANT A PARTIR D'UN MATERIAU D'IMPLANT

(30) Priorität: 05.12.2005 DE 102005058760
(43) Veröffentlichungstag der Anmeldung: 27.08.2008
(73) Patentinhaber: Aesculap AG, 78532 Tuttlingen (DE)
(72) Erfinder: LEITNER, François, F-38410 Uriage (FR)
(74) Vertreter: Hoeger, Stellrecht & Partner Patentanwälte
(86) Internationale Anmeldenummer: PCT/EP2006/068861
(87) Internationale Veröffentlichungsnummer: WO 2007/065803

(56) Entgegenhaltungen:
- JP-A- 2003 126 124
- US-A- 6 126 690
- US-A1- 2003 055 502

## Beschreibung

Die vorliegende Erfindung betrifft ein Verfahren zum Präparieren eines zum Ausfüllen eines Defekts an einem menschlichen oder tierischen Körper dienenden Implantats aus einem Implantatmaterial. Ferner betrifft die vorliegende Erfindung eine Vorrichtung zum Präparieren eines zum Ausfüllen eines Defekts an einem menschlichen oder tierischen Körper dienenden Implantats aus einem Implantatmaterial.

Das Dokument US 2003/055502 A1 stellt der nächste Stand der Technik dar. Es offenbart ein Verfahren zum Präparieren eines zum Ausfüllen eines Defekts an einem menschlichen oder tierischen Körper dienenden Implantats aus einem Implantatmaterial, bei welchem ein Defektbild des eine Defektkontur aufweisenden Defekts bereitgestellt wird. Diese Daten werden zu einem Bearbeitungswerkzeug übertragen, das über das Implantatmaterial bewegt wird, so daß es der Defektkontur folgt.

Beispielsweise zur Behandlung von durch Verletzungen entstandenen Knorpeldefekten werden Implantate benötigt, die genau der Form und der Größe des Defekts entsprechen. Derartige Implantate umfassen beispielsweise einen Träger, der mit körpereigenen Knorpelzellen des Patienten beimpft werden kann. Ein Verfahren der eingangs beschriebenen Art ist beispielsweise im deutschen Gebrauchsmuster 20 2005 005 085 beschrieben. Zur Durchführung des bekannten Verfahrens ist jedoch ein Navigationssystem erforderlich, mit dessen Hilfe eine Defektkontur des zu behandelnden Defekts mit einem navigierten Tastinstrument bestimmt wird. Dieses Verfahren ist aufwändig und kann nicht durchgeführt werden, wenn kein Navigationssystem zur Verfügung steht.

Es ist daher Aufgabe der vorliegenden Erfindung, ein Verfahren und eine Vorrichtung zum Präparieren eines zum Ausfüllen eines Defekts an einem menschlichen oder tierischen Körper dienenden Implantats aus einem Implantatmaterial so zu verbessern, dass das Implantat auf einfache Weise passgenau zum Defekt präpariert werden kann.

Diese Aufgabe wird bei einem Verfahren der eingangs beschriebenen Art erfindungsgemäß dadurch gelöst, dass ein Defektbild des eine Defektkontur aufweisenden Defekts bereitgestellt wird, auf welchem ein im oder benachbart des Defekts angeordneter erster Kalibrierkörper dargestellt ist, dass auf das oder benachbart dem zu bearbeitenden Implantatmaterial ein zweiter Kalibrierkörper angeordnet wird, welcher dem ersten Kalibrierkörper entspricht, dass ein Echtzeitbild des Implantatmaterials aufgenommen und auf einer Anzeigevorrichtung in Echtzeit dargestellt wird, dass das bereitgestellte Defektbild auf der Anzeigevorrichtung dargestellt und dem Echtzeitbild derart überlagert wird, dass der erste und der zweite Kalibrierkörper übereinander in gleicher Form und Größe dargestellt werden und dass ein Bearbeitungswerkzeug im Echtzeitbild auf der Anzeigevorrichtung dargestellt und derart über das Implantatmaterial bewegt wird, dass es der auf der Anzeigevorrichtung im Defektbild dargestellten Defektkontur folgt.

Durch die Überlagerung des Echtzeitbilds und des Defektbilds derart, dass der erste und der zweite Kalibrierkörper übereinander in gleicher Form und Größe dargestellt werden, entspricht der auf der Anzeigevorrichtung dargestellte Defekt sowohl in seiner Form als auch in seiner Größe dem aus dem Implantatmaterial zu präparierenden Implantat. Wird das Bearbeitungswerkzeug so über das Implantatmaterial bewegt, dass es in der Anzeigevorrichtung der im Defektbild dargestellten Defektkontur folgt, kann so das gewünschte Implantat in genau der Größe und Form aus dem Implantatmaterial präpariert werden, wie es dem Defekt entspricht. Der Vorteil dieses Verfahrens ist insbesondere darin zu sehen, dass kein Navigationssystem erforderlich ist. Ferner muss auch kein Echtzeitbild des Defekts zur Verfügung stehen. Dies bedeutet, dass das Verfahren weder in einem Operationssaal noch durch einen Arzt durchgeführt werden muss. Es eignet sich jedoch hervorragend, um es auch in einem Operationssaal durchzuführen, denn es ist lediglich eine Bilderzeugungsvorrichtung zur Erzeugung des Echtzeitbilds sowie eine Anzeigevorrichtung erforderlich, die ohne Weiteres in einen Sterilbereich eingebracht werden können. Dagegen ist es gerade nicht erforderlich, einen Projektor in den sterilen Bereich einzubringen, mit dem eine Defektkontur auf das zu bearbeitende Implantatmaterial übertragen werden kann. Unter einem Kalibrierkörper ist auch eine Kalibrierstruktur zu verstehen, die aus mehreren in einer festen Beziehung zueinander stehenden Teilen gebildet wird. Des Weiteren kann der erste Kalibrierkörper auch ein virtueller Kalibrierkörper sein, das heißt eine in das Defektbild eingeblendete Kalibrierstruktur oder ein Kalibrierkörper, der dem zweiten Kalibrierkörper entspricht.

Vorteilhafterweise wird ein flächiges Implantat aus einem im Wesentlichen flachen Implantatmaterial präpariert. Auf diese Weise können sowohl Knorpeldefekte als auch Hautimplantate oder dergleichen in gewünschter Weise präpariert werden. Das Verfahren eignet sich somit zum Präparieren aller Arten von Implantaten, die zum Ausfüllen eines Defekts am Körper geeignet sind.

Vorteilhaft ist es, wenn zur Aufnahme des Echtzeitbildes eine erste Bilderzeugungsvorrichtung mit einer ersten optischen Achse verwendet wird, die unter einem ersten Bildwinkel relativ zu einer vom Implantatmaterial definierten Ebene ausgerichtet wird, wenn ein Defektbild bereitgestellt wird, welches mit einer zweiten, eine zweite optische Achse aufweisenden Bilderzeugungsvorrichtung aufgenommen wurde, wobei die zweite optische Achse unter einem zweiten Bildwinkel relativ zu einer vom Defekt definierten Ebene ausgerichtet war, und wenn der erste Bildwinkel entsprechend dem zweiten Bildwinkel eingestellt wird. Auf diese Weise kann sichergestellt werden, dass sowohl der Defekt im Defektbild als auch das Implantatmaterial im Echtzeitbild auf der Anzeigevorrichtung unter dem gleichen Winkel dargestellt werden. So werden Verzerrungsfehler bei der Darstellung der beiden überlagerten Bilder auf der Anzeigevorrichtung vermieden und sichergestellt, dass auch bei einer verzerrten Darstellung des Defekts und des Implantatmaterials das zu präparierende Implantat exakt den Defekt ausfüllt.

Um Verzerrungen bei der Darstellung sowohl des Defekts als auch des Implantatmaterials zu vermeiden, ist es günstig, wenn die zweite optische Achse senkrecht oder im Wesentlichen senkrecht zu der vom Defekt definierten Ebene ausgerichtet war und wenn die erste optische Achse senkrecht oder im Wesentlichen senkrecht zu der vom Implantatmaterial definierten Ebene ausgerichtet wird.

Gemäß einer bevorzugten Variante des erfindungsgemäßen Verfahrens kann vorgesehen sein, dass ein Defektbild bereitgestellt wird, welches durch Abtasten der Defektkontur mit einem navigierten Tastinstrument aufgenommen wurde. Ein derartiges Defektbild hat den Vorteil, dass auf Grund seiner Erzeugung durch navigiertes Abtasten der Defektkontur direkt ein Maßstab bekannt ist und Abmessungen des Defekts und der Defektkontur direkt ermittelt werden können. Ferner hat ein solches Defektbild den Vorteil, dass eine virtuelle Kalibrierstruktur oder ein virtueller Kalibrierkörper in das Defektbild eingeblendet werden können, und zwar im richtigen Größenmaßstab. Sind beispielsweise Form und Größe des ersten Kalibrierkörpers bekannt, so lässt sich ein diesem entsprechender zweiter Kalibrierkörper direkt und im richtigen Größenmaßstab in das Defektbild einblenden. Dadurch wird das Anordnen eines realen Kalibrierkörpers in oder an den Defekt und damit das Einführen eines Fremdkörpers in einen menschlichen oder tierischen Körper überflüssig.

Grundsätzlich wäre es denkbar, das Defektbild als Hintergrundbild auf der Anzeigevorrichtung darzustellen. Vorteilhaft ist es jedoch, wenn das Echtzeitbild als Hintergrundbild und das Defektbild als Vordergrundbild auf der Anzeigevorrichtung dargestellt werden. In beiden Fällen kann eine Bewegung des Bearbeitungswerkzeugs optimal verfolgt werden.

Prinzipiell wäre es denkbar, dass das Defektbild und das Echtzeitbild relativ zueinander nur in ihrer Größe verändert werden, so dass die beiden Kalibrierkörper gleich groß dargestellt werden. Günstig ist es jedoch, wenn das Defektbild und das Echtzeitbild auf der Anzeigevorrichtung in Deckung gebracht werden durch Verschieben der beiden Darstellungen relativ zueinander und Ändern eines Vergrößerungsfaktors der beiden Darstellungen relativ zueinander. Auf diese einfache Weise können die beiden Kalibrierkörper auf der Anzeigevorrichtung übereinander dargestellt werden, so dass sofort erkennbar ist, ob sie dieselbe Größe und dieselbe Position aufweisen. Dadurch wird erreicht, dass ein Maßstab des Defektbilds einem Maßstab des Echtzeitbilds entspricht, so dass, ohne weitere Messvorrichtungen benutzen zu müssen, eine Kontur des Defekts 1:1 auf das Implantatmaterial übertragen werden kann.

Grundsätzlich wäre es denkbar, das Defektbild in Größe und Position so zu verändern, dass die beiden Kalibrierkörper übereinander zur Deckung gebracht werden können. Vorteilhaft ist es jedoch, wenn das Defektbild unverändert auf der Anzeigevorrichtung dargestellt wird und wenn das Echtzeitbild relativ zum Defektbild derart verschoben und vergrößert oder verkleinert wird, dass der erste Kalibrierkörper und der zweite Kalibrierkörper auf der Anzeigevorrichtung deckungsgleich dargestellt werden. Diese Vorgehensweise ist besonders einfach, da dann das bereitgestellte Defektbild nicht mehr verändert werden muss, wohingegen das Echtzeitbild, da es direkt bei der Durchführung des Verfahrens aufgenommen wird, ohne weiteres verändert werden kann, beispielsweise auch durch Einstellen eines Vergrößerungsfaktors an der ersten Bilderzeugungsvorrichtung oder Änderung einer Position derselben relativ zum Implantatmaterial.

Um zu vermeiden, dass das zu präparierende Implantat nicht optimal passgenau zum Defekt hergestellt wird, ist es günstig, wenn als erster und zweiter Kalibrierkörper jeweils ein flacher Körper verwendet wird. Dadurch können etwaige Verzerrungen bei der Überlagerung der Darstellungen der beiden Kalibrierkörper auf der Anzeigevorrichtung vermieden werden.

Günstigerweise wird ein scheibenförmiger Körper verwendet. Ein scheibenförmiger Körper hat keine besondere Vorzugsrichtung, so dass die beiden Kalibrierkörper allein durch Verschiebung in zwei zueinander senkrechten Richtungen und durch Einstellen eines Vergrößerungsfaktors deckungsgleich auf der Anzeigevorrichtung bei Überlagerung des Defektbilds und des Echtzeitbilds dargestellt werden können.

Vorteilhaft ist es, wenn als erster und zweiter Kalibrierkörper jeweils eine Kalibrierstruktur verwendet wird, die mindestens zwei in einer festen geometrischen Beziehung zueinander stehende Kalibrierelemente umfasst. Dies hat insbesondere den Vorteil, dass die Kalibrierstruktur so gewählt werden kann, dass kein Kalibrierelement direkt mit dem Implantatmaterial in Kontakt kommen kann beziehungsweise muss, was zum einen das Präparieren des Implantats aus dem Implantatmaterial behindern oder auch das Implantatmaterial selbst beschädigen könnte.

Zur Erhöhung der Genauigkeit des Verfahrens ist es günstig, wenn eine Kalibrierstruktur verwendet wird, die einen Träger umfasst, an dem zwei, drei, vier oder mehr Kalibrierkörper angeordnet sind. Insbesondere können die Kalibrierelemente weit voneinander entfernt angeordnet sein, wodurch eine Genauigkeit bei der Überlagerung des Defektbilds und des Echtzeitbilds erhöht werden kann.

Das Echtzeitbild lässt sich besonders einfach erzeugen, wenn als erste Bilderzeugungsvorrichtung eine digitale Videokamera verwendet wird.

Um das Defektbild auf einfache Weise mit einer Datenverarbeitungsvorrichtung verarbeiten zu können, ist es günstig, wenn ein mit einer Digitalkamera erzeugtes Defektbild verwendet wird. Dies ermöglicht es, das Defektbild direkt auf der Anzeigevorrichtung darzustellen und mit einem weiteren digitalen Bild, beispielsweise dem Echtzeitbild, zu überlagern.

Gemäß einer bevorzugten Variante des erfindungsgemäßen Verfahrens kann vorgesehen sein, dass vor dem Präparieren des Implantats das Defektbild mit der zweiten Bilderzeugungsvorrichtung aufgenommen wird und dass vor dem Aufnehmen des Defektbilds der zweite Kalibrierkörper im oder benachbart des Defekts angeordnet wird. Auf diese Weise kann ein gewünschtes Defektbild erzeugt werden, welches benötigt wird, um das gewünschte Implantat zu präparieren.

Um zu vermeiden, dass ein Kalibrierkörper überhaupt an den Defekt herangeführt werden muss, ist es günstig, wenn vor dem Präparieren des Implantats das Defektbild mit der zweiten Bilderzeugungsvorrichtung aufgenommen wird und wenn nach dem Aufnehmen des Defektbildes der zweite Kalibrierkörper in das Defektbild im oder benachbart des Defekts eingeblendet wird. Es wird also kein realer, sondern ein virtueller zweiter Kalibrierkörper verwendet, beispielsweise auch in Form einer Kalibrierstruktur. Der zweite Kalibrierkörper entspricht wiederum dem ersten Kalibrierkörper, wobei der zweite Kalibrierkörper in Form und Größe so gewählt und dargestellt wird, dass er einer Darstellung des ersten Kalibrierkörpers im Defektbild unter Berücksichtigung des Vergrößerungsmaßstabs des Defektbilds entspricht. Die zweite Bilderzeugungsvorrichtung kann insbesondere ein Navigationssystem in Verbindung mit einem navigierten Tastinstrument umfassen, mit dem eine Kontur, also eine Defektkontur, des Defekts abgetastet und gespeichert werden kann. Die so ermittelten Daten lassen sich in Form eines Defektbilds darstellen, beispielsweise auf einem Bildschirm.

Verzerrungen bei der Aufnahme des Defektbilds und des Defekts werden vermieden, wenn zur Aufnahme eines Defektbildes die zweite Bilderzeugungsvorrichtung derart ausgerichtet wird, dass ihre optische Achse senkrecht oder im Wesentlichen senkrecht zu einer vom Defekt definierten Ebene ausgerichtet ist.

Vorteilhafterweise wird als zweite Bilderzeugungsvorrichtung eine Digitalkamera verwendet. Diese kann beispielsweise mit einem Endoskop verbunden werden, so dass insbesondere auch Defektbilder aus dem Inneren eines menschlichen oder tierischen Körpers auch bei einem minimalinvasiven Eingriff aufgenommen werden können, beispielsweise im Rahmen einer Arthroskopie eines Kniegelenks.

Vorzugsweise wird mit dem Bearbeitungswerkzeug eine der Defektkontur entsprechende Bearbeitungsbahn auf das Implantatmaterial aufgezeichnet. Die Defektkontur ist dann auf dem Implantatmaterial zu erkennen und das zu präparierende Implantat kann dann beispielsweise aus dem Implantatmaterial durch Schneiden herausgetrennt werden.

Die Defektkontur lässt sich besonders leicht auf das Implantatmaterial übertragen, wenn als Bearbeitungswerkzeug ein Markierstift zum Markieren der Bearbeitungsbahn auf dem Implantatmaterial verwendet wird. Vorzugsweise wird ein Markierstift verwendet, mit dem eine Kontur auf das Implantat aufgezeichnet werden kann, ohne dieses zu beschädigen.

Alternativ kann es auch vorteilhaft sein, wenn mit dem Bearbeitungswerkzeug das zu präparierende Implantat aus dem Implantatmaterial ausgeschnitten wird. Es kann also, auch ohne die Bearbeitungsbahn auf dem Implantatmaterial zu markieren, das Implantat präpariert werden, wodurch ein zusätzlicher Verfahrensschritt eingespart werden kann.

Günstigerweise wird als Bearbeitungswerkzeug ein Schneidwerkzeug verwendet. Damit lässt sich das Implantat aus dem Implantatmaterial ohne Mühe heraustrennen.

Günstig ist es, wenn als Schneidwerkzeug ein Laser oder ein Skalpell verwendet werden. Derartige Schneidwerkzeuge eignen sich hervorragend um ein Implantat aus einem Implantatmaterial herauszutrennen.

Um einen Knorpeldefekt, beispielsweise in einem Kniegelenk, in gewünschter Weise behandeln zu können, ist es günstig, wenn mit dem Verfahren ein Knorpelersatzimplantat aus einem Knorpelersatzimplantatmaterial präpariert wird. Beispielsweise kann das Knorpelersatzimplantatmaterial ein Trägermaterial sein, welches mit autologen Chondrozyten beimpft wird, die nach vorheriger Entnahme aus dem Körper des Patienten im Labor gezüchtet wurden.

Die eingangs gestellte Aufgabe wird ferner erfindungsgemäß durch eine Vorrichtung der eingangs beschriebenen Art gelöst, welche eine Eingabevorrichtung zum Übertragen eines Defektbilds des eine Defektkontur aufweisenden Defekts auf die Vorrichtung umfasst, wobei auf dem Defektbild ein im oder benachbart des Defekts angeordneter erster Kalibrierkörper dargestellt ist, wobei eine erste Bilderzeugungsvorrichtung vorgesehen ist zum Aufnehmen eines Echtzeitbildes eines auf das oder benachbart dem zu bearbeitenden Implantatmaterial angeordneten zweiten Kalibrierkörpers, wobei der erste Kalibrierkörper dem zweiten Kalibrierkörper entspricht, wobei eine Anzeigevorrichtung zum Darstellen und Überlagern des Echtzeitbildes und des Defektbildes vorgesehen ist, wobei die Vorrichtung derart ausgebildet ist, dass mit der Anzeigevorrichtung der erste und der zweite Kalibrierkörper übereinander in gleicher Form und Größe darstellbar sind und wobei mit der Anzeigevorrichtung gleichzeitig ein entlang der im Defektbild dargestellten Defektkontur bewegbares Bearbeitungswerkzeug in Echtzeit darstellbar ist.

Mit der erfindungsgemäßen Vorrichtung ist es möglich, ohne Zuhilfenahme eines Navigationssystems, ein Implantatmaterial so zu bearbeiten, dass ein Implantat präparierbar ist, welches in Form und Größe passgenau zu dem auszufüllenden Defekt ist.

Gemäß einer bevorzugten Ausführungsform der Erfindung kann vorgesehen sein, dass die Vorrichtung eine Datenverarbeitungseinrichtung umfasst und dass die Datenverarbeitungseinrichtung mit der Eingabevorrichtung und mit der ersten Bilderzeugungsvorrichtung zusammenwirkend ausgebildet ist. Beispielsweise kann die Datenverarbeitungseinrichtung in Form eines Computers ausgebildet sein, welcher Daten auf einer Anzeigevorrichtung, beispielsweise einem Monitor, darstellen kann. Die Eingabevorrichtung kann insbesondere in Form eines Lesegeräts für Speichermedien ausgebildet sein, beispielsweise in Form eines CD-Laufwerks oder einem Speicherkartenlesegeräts für Speicherkarten, wie sie beispielsweise bei Digitalkameras Verwendung finden. Alternativ wäre es auch denkbar, die Eingabevorrichtung in Form eines digitalen Eingangs an der Datenverarbeitungsvorrichtung vorzusehen, um direkt Signale von einer digitalen Kamera einzulesen.

Vorteilhafterweise ist die Datenverarbeitungsvorrichtung derart ausgebildet, dass das Defektbild und/oder das Echtzeitbild auf der Anzeigevorrichtung in ihrer Größe und Position veränderbar darstellbar sind. Beispielsweise kann die Datenverarbeitungsvorrichtung mit einer entsprechenden Software ausgestattet sein, mit der die zur Veränderung von Größe und Position eines Bildes erforderliche Bildbearbeitung durchgeführt werden kann.

Günstig ist es, wenn mit der Vorrichtung eines der oben beschriebenen erfindungsgemäßen Verfahren durchführbar ist.

Die nachfolgende Beschreibung einer bevorzugte Ausführungsform einer Erfindung dient im Zusammenhang mit der Zeichnung der näheren Erläuterung. Es zeigen:
- Figur 1:: eine schematische Darstellung bei der Aufnahme eines Defekt- bilds;
- Figur 2:: eine beispielhafte Darstellung eines Defektbilds und eines Echtzeitbilds jeweils mit Kalibrierkörper;
- Figur 3:: eine schematische Darstellung der Überlagerung und Kalibrierung des Defektbilds und des Echtzeitbilds; und
- Figur 4:: eine schematische Darstellung bei der Bearbeitung des Implantatmaterials zum Präparieren des gewünschten Implantats.
- Figur 5:: eine weitere schematische Darstellung einer Variante des Verfahrens zur Bearbeitung des Implantatmaterials zum Präparieren des gewünschten Implantats.

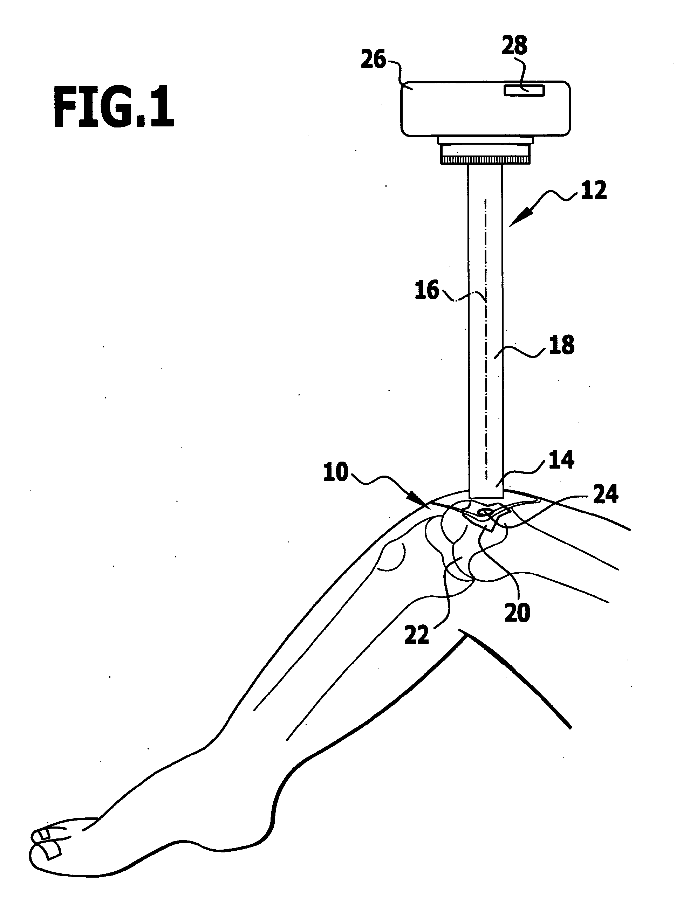

Im Zusammenhang mit den Figuren 1 bis 4 werden nachfolgend das oben beschriebene erfindungsgemäße Verfahren sowie die erfindungsgemäße Vorrichtung anhand eines bevorzugten Ausführungsbeispiels näher erläutert.

In Figur 1 ist schematisch eine Anordnung dargestellt, wie zum Beispiel im Rahmen eines minimalinvasiven Eingriffs, und zwar in Form eines arthroskopischen Eingriffs, in ein Kniegelenk 10 eines menschlichen Körpers ein Defektbild aufgenommen wird. Hierzu wird vorzugsweise ein Endoskop 12 nach Eröffnen des Kniegelenks 10 mit einem distalen Ende 14 in das eröffnete Kniegelenk 10 ein- und ein eine optische Achse 16 definierender, langgestreckter Schaft 18 des Endoskops 12 mit dem Ende 14 bis nahe an einem Defekt 20 eines Gelenkknorpels 22 herangeführt. Vor der Aufnahme des Defekts wird ein Kalibrierkörper in Form einer flachen Scheibe 24 im Defekt 20 abgelegt. Ausgerichtet mit der optischen Achse 16 des Schafts 18 wird eine Bilderzeugungsvorrichtung in Form einer Digitalkamera 26 am proximalen Ende des Schafts 18 des Endoskops 12 angeordnet. Im Inneren des Schafts 18 ist entweder ein nicht dargestellter Lichtleiter oder eine nicht dargestellte Optik angeordnet, mittels der es möglich ist, mit der Digitalkamera 26 ein Defektbild 30 des Defekts 20 aufzunehmen.

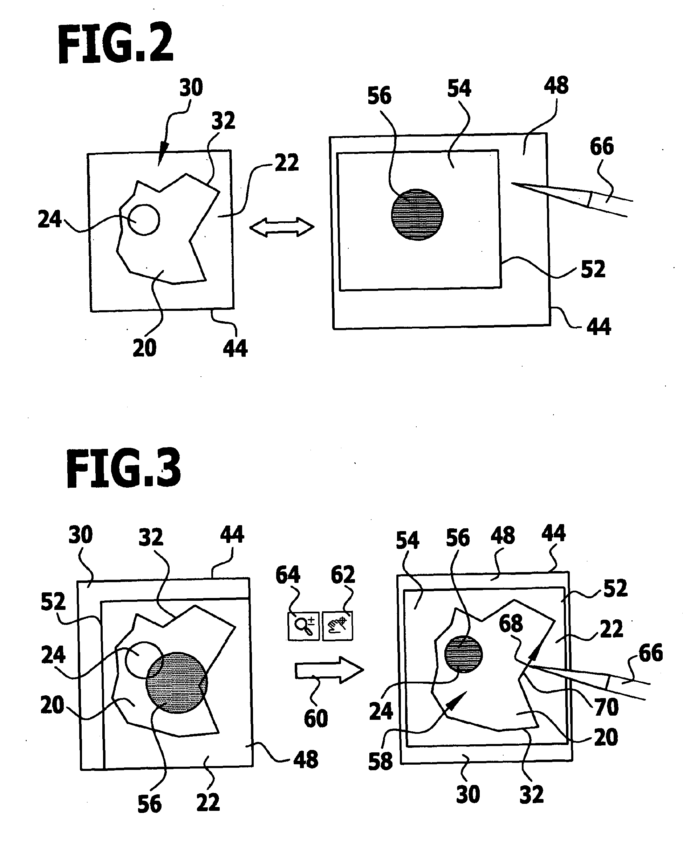

Das Defektbild 30 ist in Figur 2 links dargestellt und zeigt sowohl den Defekt 20 als auch die innerhalb des Defekts 20 angeordnete Scheibe 24. Eine Defektkontur 32, also eine Grenzlinie zwischen degeneriertem Knorpelgewebe und erhaltenem Gelenkknorpel 22 ist gut zu erkennen.

Das Defektbild 30 ist in einem Speicher 28 der Digitalkamera 26 abgelegt, beispielsweise einer Speicherkarte, die in einen Kartenleseschacht 34 eines eine Datenverarbeitungsvorrichtung bildenden Computers 36 eingeführt werden und von der dann das gespeicherte Defektbild 30 auf den Computer 36 übertragen werden kann. Der Computer 36 ist Teil einer insgesamt mit dem Bezugszeichen 38 versehenen Bearbeitungsvorrichtung zum Präparieren eines Implantats aus einem Implantatmaterial. Üblicherweise ist der Computer 36 mit einer Tastatur 40 und einer Maus 42 verbunden, über die eine Bedienperson Programme starten und bedienen kann. Ein als Anzeigevorrichtung dienender Bildschirm 44 ist ebenfalls mit dem Computer 36 verbunden und dient zur Darstellung des Defektbilds 30.

Des Weiteren ist eine digitale Videokamera 46 als weitere Bilderzeugungsvorrichtung mit dem Computer 36 verbunden, mit der ein Echtzeitbild aufgenommen und mittels des Computers 36 auf dem Bildschirm 44 dargestellt werden kann.

Zum Präparieren eines Implantats, welches in den Defekt 20 eingesetzt werden kann, um diesen passgenau auszufüllen und so eine Regeneration des geschädigten Knorpelgewebes zu ermöglichen, wird die Videokamera 46 mit ihrer optischen Achse 50 im Wesentlichen senkrecht zu einer von einem in einer flachen Wanne 52 gelagerten Implantatmaterial 54 definierten Ebene ausgerichtet. Auf das Implantatmaterial 54 wird ein weiterer Kalibrierkörper in Form einer Kalibrierscheibe 56 gelegt. Die Kalibrierscheibe 56 und die Scheibe 24 sind identisch ausgebildet, sowohl in ihrem Durchmesser als auch in ihrer Höhe. Das so erzeugte Echtzeitbild 48 wird auf dem Bildschirm 44 dargestellt, wie dies in Figur 2 rechts zu sehen ist.

Bei der Aufnahme des Echtzeitbilds 48 wird darauf geachtet, dass die optische Achse 50 relativ zu einer vom Implantatmaterial 54 definierten Ebene unter demselben Winkel ausgerichtet wird wie die optische Achse 16 bei der Aufnahme des Defektbilds 30, das heißt ebenso wie die optische Achse relativ zu einer vom Defekt 20 definierten Ebene. Vorzugsweise sind beide optischen Achsen 16 und 50 jeweils senkrecht sowohl zum Implantatmaterial 54 als auch zum Defekt 20 ausgerichtet.

Da bei der Anordnung der Videokamera 46 nicht darauf geachtet wird, in welchem Abstand diese vom Implantatmaterial 54 positioniert ist, stimmen die Darstellungen des Defektbilds 30 und des Echtzeitbilds 48 in ihrem Größenmaßstab normalerweise nicht überein. Dies zeigt sich darin, dass die in Form und Größe mit der Kalibrierscheibe 56 identische Scheibe 24 auf dem Bildschirm 44 anders dargestellt wird, nämlich, wie beispielhaft in Figur 2 dargestellt, kleiner.

Werden das Defektbild 30 und das Echtzeitbild 48 auf dem Bildschirm 44 überlagert, wie dies in Figur 3 links dargestellt ist, dann stimmen weder Position noch Größe der Scheibe 24 und der Kalibrierscheibe 56 überein. Ob dabei das Echtzeitbild 48 oder das Defektbild 30 im Vordergrund oder im Hintergrund auf dem Bildschirm 44 dargestellt werden, spielt grundsätzlich keine Rolle.

Um ein Implantat 58 zu präparieren, das in Form und Größe dem Defekt 20 entspricht, müssen das Defektbild 30 und das Echtzeitbild 48 aneinander angepasst werden, und zwar derart, dass zumindest die Größe der Scheibe 24 mit der Größe der Kalibrierscheibe 56 übereinstimmt. Zwingend überlappen müssen sie sich nicht. Dies lässt sich jedoch besonders einfach dadurch erreichen, dass die Scheibe 24 und die Kalibrierscheibe 56 zur Deckung gebracht werden. Dies wird mittels eines Justiervorgangs vorgenommen, der symbolisch mit dem Pfeil 60 in Figur 3 bezeichnet ist. Zum Beispiel kann das Echtzeitbild 48 in zwei zueinander senkrechten Richtungen verschoben werden, so dass durch diese Verschiebung 62 auf dem Bildschirm 44 die Scheibe 24 die Kalibrierscheibe 56 so überlagert, dass ihre Mittelpunkte übereinander liegen. Des Weiteren kann durch eine Veränderung eines Vergrößerungsfaktors 64 einer Darstellung des Echtzeitbilds 48 auf dem Bildschirm 44 die Größe der Kalibrierscheibe 56 so verändert werden, dass sie mit der Größe der Scheibe 24 übereinstimmt. Der Justiervorgang 60 ist abgeschlossen, wenn auf dem Bildschirm 44 nur noch ein durch die Scheibe 24 und die Kalibrierscheibe 56 gebildeter Kreis zu sehen ist. Durch den Justiervorgang 60 wird das Implantatmaterial 54 auf dem Bildschirm 44 im selben Maßstab dargestellt wie der Defekt 20.

Durch die in Figur 3 dargestellte, optimierte Darstellung vom Echtzeitbild 48 und Defektbild 30 auf dem Bildschirm 44 ist nun alles vorbereitet, um das Implantat 58 aus dem Implantatmaterial 54 zu präparieren. Hierzu wird ein Bearbeitungswerkzeug 66, beispielsweise ein Markierstift oder ein Skalpell verwendet. Kommt ein Markierstift zum Einsatz, wird dieser mit seiner Spitze 86 so über das Implantatmaterial 54 bewegt, dass die Darstellung des Bearbeitungswerkzeugs 66 im Echtzeitbild 48 auf dem Bildschirm 44 der Defektkontur 32 folgt. Es wird so eine Bewegungsbahn 70 auf das Implantatmaterial 54 aufgezeichnet, die in Form und Größe der Defektkontur 32 entspricht. Hierzu kann entweder eine Bedienperson das Bearbeitungswerkzeug 66 so über das Implantatmaterial 54 bewegen, dass die Person nur unter Betrachtung des Bildschirms 44 die Spitze 68 des Bearbeitungswerkzeugs 66 der Defektkontur 32 folgend über das Implantatmaterial 54 bewegt. Alternativ könnte auch eine Vorrichtung vorgesehen sein, die das Bearbeitungswerkzeug 66 über das Implantatmaterial 54 bewegt, wobei eine Bewegung dieser Vorrichtung in Abhängigkeit der Position des Bearbeitungswerkzeugs 66 im Echtzeitbild 48 gesteuert und geregelt wird, und zwar so, dass das Bearbeitungswerkzeug 66 stets der Defektkontur 32 folgt. Es wäre dann möglich, das Implantat 58 vollautomatisch aus dem Implantatmaterial 54 zu präparieren.

Wie bereits erwähnt kann das Bearbeitungswerkzeug 66 auch in Form eines Schneidwerkzeugs, beispielsweise eines Skalpells ausgebildet sein, so dass eine Bedienperson oder eine entsprechend geeignete Vorrichtung direkt das Implantat 58 aus dem Implantatmaterial 54 in der beschriebenen Weise heraustrennen können.

Das beschriebene Verfahren kann von jeder Person durchgeführt werden, da es nicht zwingend in einem Operationssaal durchgeführt werden muss. Wird ein Defektbild 30 bereitgestellt, kann an jedem beliebigen Ort das Implantat 58 aus dem Implantatmaterial 54 präpariert werden.

Alternativ kann der Justiervorgang 60 so durchgeführt werden, dass das Echtzeitbild 48 unverändert auf dem Bildschirm 44 dargestellt wird und durch Verschieben und Einstellen eines Vergrößerungsfaktors das Defektbild 30 dem Echtzeitbild so überlagert wird, bis die Scheibe 24 und die Kalibrierscheibe 56 deckungsgleich übereinander dargestellt werden.

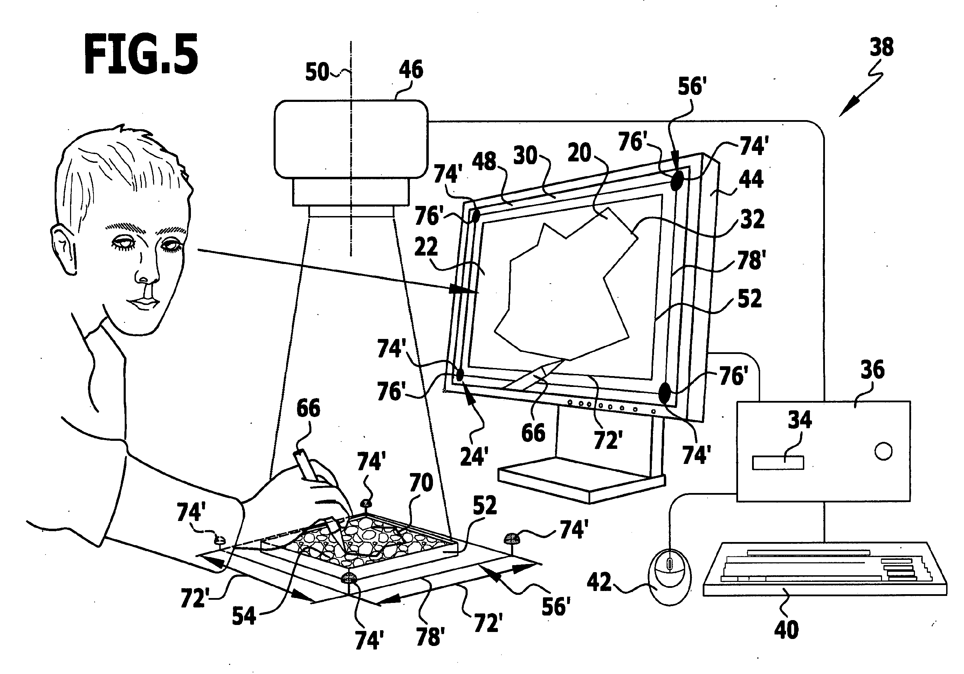

Im Zusammenhang mit Figur 5 wird nachfolgend eine gegenüber dem oben beschriebenen Verfahren etwas modifizierte zweite Variante des erfindungsgemäßen Verfahrens zusammen mit einem zweiten Ausführungsbeispiel einer erfindungsgemäßen Vorrichtung näher erläutert.

Die zweite Variante des erfindungsgemäßen Verfahrens unterscheidet sich von der oben beschriebenen Variante dadurch, dass zur Aufnahme des Defektbilds kein Kalibrierkörper und auch keine Kalibrierstruktur im Defekt 20 abgelegt werden. Statt dessen wird ein Kalibrierkörper in Form einer virtuellen Kalibrierstruktur 24' nach Aufnahme des Defektbildes 30 oder beim Bearbeiten des Implantatmaterials 54 direkt in das Defektbild 30 eingeblendet. Die Kalibrierstruktur 24' umfasst im vorliegenden Fall vier in einem Quadrat angeordnete ausgefüllte Punkte. Anstelle der einen zweiten Kalibrierkörper bildenden Kalibrierscheibe 56 wird eine reale Kalibrierstruktur 56' verwendet, die gebildet wird durch einen quadratischen Rahmen 78', von dem vier identische Kalibrierscheiben 74' aus der vom Rahmen 78' definierten Ebene vorstehen, wobei eine Seitenlänge 72' des Rahmens 78' einen vorgegebenen Wert aufweist, beispielsweise eine Länge von 10 cm.

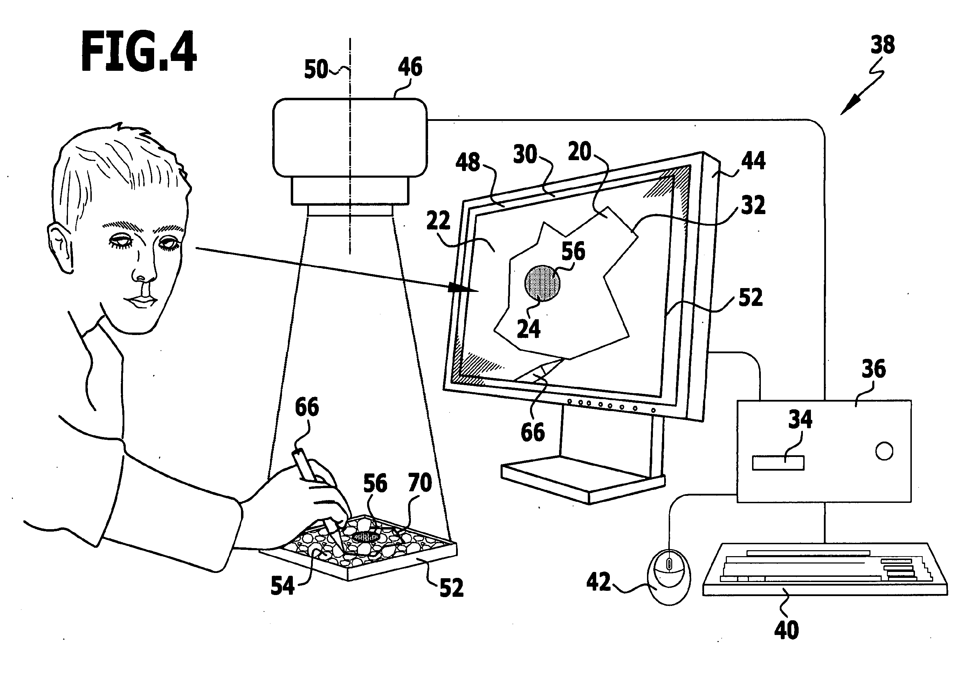

Die virtuelle Kalibrierstruktur 24' wird bei bekannter Auflösung und bei bekannter Skalierung des Defektbildes 30 derart in dieses eingeblendet, dass vier in Form einer quadratischen Struktur angeordnete virtuelle Kalibrierscheiben 76' zu sehen sind, deren dargestellte Abstände in Relation zum Defekt 20 der Seitenlänge 72' entsprechen. Da ein Vergrößerungsfaktor des Defektbilds 30 nicht zwingend einem Vergrößerungsfaktor des Echtzeitbilds 48 entsprechen muss, kann die tatsächliche Größe der im Defektbild 30 eingeblendeten Kalibrierstruktur 24' von der Größe der im Echtzeitbild 48 dargestellten Kalibrierstruktur 56' abweichen. Durch entsprechende Veränderung eines Vergrößerungsfaktors 64 einer Darstellung des Echtzeitbilds 48 auf dem Bildschirm 44 sowie entsprechender Positionsänderung des Echtzeitbilds 48 und des Defektbilds 30 relativ zueinander kann die Kalibrierstruktur 24' der Kalibrierstruktur 56' so überlagert werden, dass nur noch eine einzige Kalibrierstruktur 24' beziehungsweise 56' für einen Beobachter sichtbar ist. Die Defektkontur 32 ist dann, wie bereits im Zusammenhang mit Figur 4 beschrieben, virtuell auf dem Bildschirm 44 auf das Implantatmaterial 54 übertragen, dass mit einem Bearbeitungswerkzeug 66, dessen Bewegung in dem auf dem Bildschirm 44 dargestellten Echtzeitbild 48 verfolgt und so das Implantat 58 präpariert werden kann, entweder von Hand oder vollautomatisch.

Alternativ ist es auch möglich, das Defektbild zu erzeugen durch Abtasten der Defektkontur 32 mit einem navigierten Tastinstrument, dessen Bewegung mit einem Navigationssystem nachverfolgt werden kann. Dies hat insbesondere den Vorteil, dass keine optische Bilderzeugungsvorrichtung zur Aufnahme des Defektbilds erforderlich ist und zudem die Kalibrierstruktur 24' virtuell in das Defektbild 30 im richtigen Maßstab eingeblendet werden kann, da beim navigierten Abtasten der Defektkontur 32 auch der Vergrößerungsmaßstab direkt von einem Navigationssystem ermittelt und bereitgestellt werden kann.

## Patentansprüche

1. Verfahren zum Präparieren eines zum Ausfüllen eines Defekts (20) an einem menschlichen oder tierischen Körper dienenden Implantats (58) aus einem Implantatmaterial (54), bei welchem ein Defektbild (30) des eine Defektkontur (32) aufweisenden Defekts (20) bereitgestellte wird, auf welchem ein im oder benachbart des Defekts (20) angeordneter erster Kalibrierkörper (24) dargestellt ist, wobei auf das oder benachbart dem zu bearbeitenden Implantatmaterial (54) ein zweiter Kalibrierkörper (56) angeordnet wird, welcher dem ersten Kalibrierkörper (24) entspricht, wobei ein Echtzeitbild (48) des Implantatmaterials (54) aufgenommen und auf einer Anzeigevorrichtung (44) in Echtzeit dargestellt wird, wobei das bereitgestellte Defektbild (30) auf der Anzeigevorrichtung (44) dargestellt und dem Echtzeitbild (48) derart überlagert wird, dass der erste und der zweite Kalibrierkörper (24, 56) übereinander in gleicher Form und Größe dargestellt werden und wobei ein Bearbeitungswerkzeug (66) im Echtzeitbild (48) auf der Anzeigevorrichtung (44) dargestellt und derart über das Implantatmaterial (54) bewegt wird, dass es der auf der Anzeigevorrichtung (44) im Defektbild (30) dargestellten Defektkontur (32) folgt.

2. Verfahren nach einem der voranstehenden Ansprüche, **dadurch gekennzeichnet, dass** zur Aufnahme des Echtzeitbilds (48) eine erste Bilderzeugungsvorrichtung (46) mit einer ersten optischen Achse (50) verwendet wird, die unter einem ersten Bildwinkel relativ zu einer vom Implantatmaterial (54) definierten Ebene ausgerichtet wird, dass ein Defektbild (30) bereitgestellt wird, welches mit einer zweiten, eine zweite optische Achse (16) aufweisenden Bilderzeugungsvorrichtung (26) aufgenommen wurde, wobei die zweite optische Achse (16) unter einem zweiten Bildwinkel relativ zu einer vom Defekt (20) definierten Ebene ausgerichtet war, und dass der erste Bildwinkel entsprechend dem zweiten Bildwinkel eingestellt wird.

3. Verfahren nach Anspruch 1, **dadurch gekennzeichnet, dass** ein Defektbild (30) bereitgestellt wird, welches durch Abtasten der Defektkontur (32) mit einem navigierten Tastinstrument aufgenommen wurde.

4. Verfahren nach einem der voranstehenden Ansprüche, **dadurch gekennzeichnet, dass** das Defektbild (30) und das Echtzeitbild (48) auf der Anzeigevorrichtung (44) in Deckung gebracht werden durch Verschieben der beiden Darstellungen relativ zueinander und Ändern eines Vergrößerungsfaktors der beiden Darstellungen relativ zueinander.

5. Verfahren nach einem der voranstehenden Ansprüche, **dadurch gekennzeichnet, dass** als erster und zweiter Kalibrierkörper (24', 56') jeweils eine Kalibrierstruktur (24', 56') verwendet wird, die mindestens zwei in einer festen geometrischen Beziehung zueinander stehende Kalibrierelemente (76', 74') umfasst.

6. Verfahren nach einem der Ansprüche 2 bis 5, **dadurch gekennzeichnet, dass** vor dem Präparieren des Implantats (58) das Defektbild (30) mit der zweiten Bilderzeugungsvorrichtung (26) aufgenommen wird und dass vor dem Aufnehmen des Defektbildes (30) der zweite Kalibrierkörper (24) im oder benachbart des Defekts (20) angeordnet wird.

7. Verfahren nach einem der Ansprüche 2 bis 5, **dadurch gekennzeichnet, dass** vor dem Präparieren des Implantats (58) das Defektbild (30) mit der zweiten Bilderzeugungsvorrichtung (26) aufgenommen wird und dass nach dem Aufnehmen des Defektbildes (30) der zweite Kalibrierkörper (24) in das Defektbild im oder benachbart des Defekts (20) eingeblendet wird.

8. Verfahren nach Anspruch 6 oder 7, **dadurch gekennzeichnet, dass** zur Aufnahme des Defektbildes (30) die zweite Bilderzeugungsvorrichtung (26) derart ausgerichtet wird, dass ihre optische Achse (16) senkrecht oder im Wesentlichen senkrecht zu einer vom Defekt (20) definierten Ebene ausgerichtet ist.

9. Verfahren nach einem der voranstehenden Ansprüche, **dadurch gekennzeichnet, dass** mit dem Bearbeitungswerkzeug (66) eine der Defektkontur (32) entsprechende Bearbeitungsbahn (70) auf das Implantatmaterial (54) aufgezeichnet wird.

10. Verfahren nach einem der voranstehenden Ansprüche, **dadurch gekennzeichnet, dass** mit dem Bearbeitungswerkzeug (66) das zu präparierende Implantat (58) aus dem Implantatmaterial (54) ausgeschnitten wird.

11. Verfahren nach einem der voranstehenden Ansprüche, **dadurch gekennzeichnet, dass** mit dem Verfahren ein Knorpelersatzimplantat (58) aus einem Knorpelersatzimplantatmaterial (54) präpariert wird.

12. Vorrichtung (38) zum Präparieren eines zum Ausfüllen eines Defekts (20) an einem menschlichen oder tierischen Körper dienenden Implantats (58) aus einem Implantatmaterial (54), mit einer Eingabevorrichtung (34) zum Übertragen eines Defektbilds (30) des eine Defektkontur (32) aufweisenden Defekts (20) auf die Vorrichtung (38), wobei auf dem Defektbild (30) ein im oder benachbart des Defekts (20) angeordneter erster Kalibrierkörper (24, 24') dargestellt ist, wobei eine erste Bilderzeugungsvorrichtung (46) vorgesehen ist zum Aufnehmen eines Echtzeitbildes (48) eines auf das oder benachbart dem zu bearbeitenden Implantatmaterial (54) angeordneten zweiten Kalibrierkörpers (56, 56'), wobei der erste Kalibrierkörper (24, 24') dem zweiten Kalibrierkörper (56, 56') entspricht, wobei eine Anzeigevorrichtung (44) zum Darstellen und Überlagern des Echtzeitbildes (48) und des Defektbildes (30) vorgesehen ist, wobei die Vorrichtung (38) derart ausgebildet ist, dass mit der Anzeigevorrichtung (44) der erste und der zweite Kalibrierkörper (24, 56; 24', 56') übereinander in gleicher Form und Größe darstellbar sind und wobei mit der Anzeigevorrichtung (44) gleichzeitig ein entlang der im Defektbild (30) dargestellten Defektkontur (32) bewegbares Bearbeitungswerkzeug (66) in Echtzeit darstellbar ist.

13. Vorrichtung nach Anspruch 12, **dadurch gekennzeichnet, dass** die Vorrichtung (38) eine Datenverarbeitungseinrichtung (36) umfasst und dass die Datenverarbeitungseinrichtung (36) mit der Eingabevorrichtung (34) und mit der ersten Bilderzeugungsvorrichtung (46) zusammenwirkend ausgebildet ist.

14. Vorrichtung nach Anspruch 13, **dadurch gekennzeichnet, dass** die Datenverarbeitungsvorrichtung (36) derart ausgebildet ist, dass das Defektbild (30) und/oder das Echtzeitbild (48) auf der Anzeigevorrichtung (44) in ihrer Größe und Position veränderbar darstellbar sind.

15. Vorrichtung nach einem der Ansprüche 12 bis 14, **dadurch gekennzeichnet, dass** mit der Vorrichtung (38) ein Verfahren nach einem der Ansprüche 1 bis 11 durchführbar ist.

## Claims

1. Method for preparing an implant (58) from an implant material (54), the implant serving to fill a defect (20) in a human or animal body, wherein a defect image (30) of the defect (20) having a defect contour (32) is made available, a first calibration member (24) arranged in or adjacent to the defect (20) being displayed in the image, wherein a second calibration member (56) is arranged on or adjacent to the implant material (54) to be processed, the second calibration member corresponding to the first calibration member (24), wherein a real-time image (48) of the implant material (54) is taken and displayed in real time on a display device (44), wherein the defect image (30) made available is displayed on the display device (44) and superimposed on the real-time image (48) in such a way that the first and the second calibration members (24, 56) are displayed one on top of the other in the same shape and size, and wherein a processing tool (66) is displayed in the real-time image (48) on the display device (44) and moved over the implant material (54) in such a way that it follows the defect contour (32) displayed in the defect image (30) on the display device (44).

2. Method in accordance with any one of the preceding claims, **characterized in that** a first image generating device (46) with a first optical axis (50) is used for taking the real-time image (48), the axis being aligned at a first image angle relative to a plane defined by the implant material (54), **in that** a defect image (30) is made available, which has been taken with a second image generating device (26) having a second optical axis (16), the second optical axis (16) having been aligned at a second image angle relative to a plane defined by the defect (20), and **in that** first image angle is set in accordance with the second image angle.

3. Method in accordance with claim 1, **characterized in that** a defect image (30) is made available, which has been taken by scanning the defect contour (32) with a navigated palpation instrument.

4. Method in accordance with any one of the preceding claims, **characterized in that** the defect image (30) and the real-time image (48) are brought into coincidence on the display device (44) by moving the two representations relative to each other and altering an enlarging factor of the two representations relative to each other.

5. Method in accordance with any one of the preceding claims, **characterized in that** a calibration structure (24', 56') comprising at least two calibration elements (76', 74') in a fixed, geometric relation to each other is used as first and second calibration member (24', 56'), respectively.

6. Method in accordance with any one of claims 2 to 5, **characterized in that** prior to preparing the implant (58) the defect image (30) is taken with the second image generating device (26), and **in that** prior to taking the defect image (30) the second calibration member (24) is arranged in or adjacent to the defect (20).

7. Method in accordance with any one of claims 2 to 5, **characterized in that** prior to preparing the implant (58) the defect image (30) is taken with the second image generating device (26), and **in that** after taking the defect image (30) the second calibration member (24) is superimposed onto the defect image in or adjacent to the defect (20).

8. Method in accordance with claim 6 or 7, **characterized in that** in order to take the defect image (30) the second image generating device (26) is aligned in such a way that its optical axis (16) is aligned perpendicularly or substantially perpendicularly to a plane defined by the defect (20).

9. Method in accordance with any one of the preceding claims, **characterized in that** a processing path (70) corresponding to the defect contour (32) is drawn onto the implant material (54) with the processing tool (66).

10. Method in accordance with any one of the preceding claims, **characterized in that** the implant (58) to be prepared is cut out of the implant material (54) with the processing tool (66).

11. Method in accordance with any one of the preceding claims, **characterized in that** a cartilage replacement implant (58) is prepared from a cartilage replacement implant material (54) with the method.

12. Device (38) for preparing an implant (58) from an implant material (54), the implant serving to fill a defect (20) in a human or animal body, comprising an input device (34) for transferring a defect image (30) of the defect (20) having a defect contour (32) to the device (38), a first calibration member (24, 24') arranged in or adjacent to the defect (20) being displayed on the defect image (30), a first image generating device (46) being provided for taking a real-time image (48) of a second calibration member (56, 56') arranged on or adjacent to the implant material (54) to be processed, the first calibration member (24, 24') corresponding to the second calibration member (56, 56'), a display device (44) being provided for displaying and superimposing the real-time image (48) and the defect image (30), the device (38) being designed such that the first and the second calibration members (24, 56; 24', 56') can be displayed with the display device (44) one on top of the other in the same shape and size, and a processing tool (66) movable along the defect contour (32) displayed in the defect image (30) being able to be displayed at the same time with the display device (44) in real time.

13. Device in accordance with claim 12, **characterized in that** the device (38) comprises a data processing device (36), and **in that** the data processing device (36) is designed to interact with the input device (34) and with the first image generating device (46).

14. Device in accordance with claim 13, **characterized in that** the data processing device (36) is designed such that the defect image (30) and/or the real-time image (48) can be displayed on the display device (44) so as to be alterable in their size and position.

15. Device in accordance with any one of claims 12 to 14, **characterized in that** a method in accordance with any one of claims 1 to 11 can be performed with the device (38).

## Revendications

1. Procédé de préparation d'un implant (58) en un matériau d'implant (54), qui est destiné à combler un déficit ou un défaut (20) sur un corps humain ou animal, procédé selon lequel
on fournit une image de défaut (30) du déficit ou défaut (20) présentant un contour de défaut (32), sur laquelle est représenté un premier corps de calibrage (24) agencé dans le défaut (20) ou au voisinage de celui-ci,
on agence sur le matériau d'implant (54) à façonner ou au voisinage de celui-ci, un deuxième corps de calibrage (56), qui correspond au premier corps de calibrage (24), on relève une image en temps réel (48) du matériau d'implant (54) et on la représente en temps réel sur un dispositif d'affichage ou de visualisation (44),
on représente l'image de défaut (30) fournie sur le dispositif de visualisation (44) et on la superpose à l'image en temps réel (48) de façon telle, que le premier et le deuxième corps de calibrage (24, 56) soient représentés de manière superposée selon une même forme et une même grandeur, et
on représente un outil de façonnage (66) dans l'image en temps réel (48) sur le dispositif de visualisation (44) et on le déplace par-dessus le matériau d'implant (54) de façon à ce qu'il suive le contour de défaut (32) représenté dans l'image de défaut (30) sur le dispositif de visualisation (44).

2. Procédé selon la revendication précédente, **caractérisé en ce que** pour relever l'image en temps réel (48), on utilise un premier dispositif de production d'image (46) présentant un premier axe optique (50) que l'on oriente sous un premier angle d'image par rapport à un plan défini par le matériau d'implant (54), **en ce que** l'on fournit une image de défaut (30) qui a été relevée à l'aide d'un deuxième dispositif de production d'image (26) présentant un deuxième axe optique (16), le deuxième axe optique (16) ayant été orienté sous un deuxième angle d'image par rapport à un plan défini par le défaut (20), et **en ce que** l'on règle le premier angle d'image de manière à correspondre au deuxième angle d'image.

3. Procédé selon la revendication 1, **caractérisé en ce que** l'on fournit une image de défaut (30) qui a été relevée par palpage du contour de défaut (32) à l'aide d'un instrument de palpage déplacé par navigation.

4. Procédé selon l'une des revendications précédentes, **caractérisé en ce qu'**on amène l'image de défaut (30) et l'image en temps réel (48) en recouvrement réciproque sur le dispositif de visualisation ou d'affichage (44), par déplacement ou translation des deux représentations l'une par rapport à l'autre et modification d'un facteur d'agrandissement des deux représentations l'une par rapport à l'autre.

5. Procédé selon l'une des revendications précédentes, **caractérisé en ce que** l'on utilise en guise de premier et de deuxième corps de calibrage (24', 56') respectivement une structure de calibrage (24', 56'), qui comporte au moins deux éléments de calibrage (76', 74') se trouvant réciproquement dans une relation géométrique fixe.

6. Procédé selon l'une des revendications 2 à 5, **caractérisé en ce qu'**avant la préparation de l'implant (58), on relève l'image de défaut (30) à l'aide du deuxième dispositif de production d'image (26), et **en ce qu'**avant de relever l'image de défaut (30), on agence le deuxième corps de calibrage (24) sur le défaut (20) ou au voisinage de celui-ci.

7. Procédé selon l'une des revendications 2 à 5, **caractérisé en ce qu'**avant la préparation de l'implant (58), on relève l'image de défaut (30) à l'aide du deuxième dispositif de production d'image (26), et **en ce qu'**après avoir relevé l'image de défaut (30), on produit la surimpression du deuxième corps de calibrage (24) dans l'image de défaut, sur le défaut (20) ou au voisinage de celui-ci.

8. Procédé selon la revendication 6 ou la revendication 7, **caractérisé en ce que** pour relever l'image de défaut (30), on oriente le deuxième dispositif de production d'image (26) de façon à ce que son axe optique (16) soit orienté perpendiculairement ou sensiblement de manière perpendiculaire à un plan défini par le défaut (20).

9. Procédé selon l'une des revendications précédentes, **caractérisé en ce qu'**à l'aide de l'outil de façonnage (66) on dessine sur le matériau d'implant (54) une trajectoire de façonnage (70) correspondant au contour de défaut (32).

10. Procédé selon l'une des revendications précédentes, **caractérisé en ce qu'**à l'aide de l'outil de façonnage (66) on découpe l'implant (58) à préparer, dans le matériau d'implant (54).

11. Procédé selon l'une des revendications précédentes, **caractérisé en ce qu'**à l'aide du procédé, on prépare un implant de substitution de cartilage (58) à partir d'un matériau d'implant de substitution de cartilage (54).

12. Dispositif (38) de préparation d'un implant (58) en un matériau d'implant (54), qui est destiné à combler un déficit ou un défaut (20) sur un corps humain ou animal, comprenant un dispositif d'introduction (34) pour transférer au dispositif (38) une image de défaut (30) du défaut (20) présentant un contour de défaut (32), dispositif dans lequel
sur l'image de défaut (30) est représenté un premier corps de calibrage (24, 24') agencé dans le défaut (20) ou au voisinage de celui-ci,
il est prévu un premier dispositif de production d'image (46) pour relever une image en temps réel (48) d'un deuxième corps de calibrage (56, 56') agencé sur le matériau d'implant (54) à façonner ou au voisinage de celui-ci, le premier corps de calibrage (24, 24') correspondant au deuxième corps de calibrage (56, 56'), il est prévu un dispositif d'affichage ou de visualisation (44) pour représenter et superposer l'image en temps réel (48) et l'image de défaut (30),
le dispositif (38) est conçu de façon telle qu'à l'aide du dispositif de visualisation (44) le premier et le deuxième corps de calibrage (24, 56, 24', 56') puissent être représentés de manière superposée selon une même forme et une même grandeur, et
il est possible de représenter simultanément en temps réel, à l'aide du dispositif de visualisation (44), un outil de façonnage (66) pouvant être déplacé le long du contour de défaut (32) représenté sur l'image de défaut (30).

13. Dispositif selon la revendication 12, **caractérisé en ce que** le dispositif (38) comprend un système informatique de traitement de données (36), et **en ce que** le système informatique de traitement de données (36) est réalisé de manière à interagir avec le dispositif d'introduction (34) et avec le premier dispositif de production d' image (46).

14. Dispositif selon la revendication 13, **caractérisé en ce que** le système informatique de traitement de données (36) est conçu de façon à pouvoir représenter sur le dispositif de visualisation (44), l'image de défaut (30) et/ou l'image en temps réel (48) de manière variable quant à leur grandeur et leur position.

15. Dispositif selon l'une des revendications 12 à 14, **caractérisé en ce qu'**à l'aide du dispositif (38), il est possible de mettre en oeuvre un procédé selon l'une des revendications 1 à 11.
